(19) Europäisches Patentamt / European Patent Office / Office européen des brevets

(11) **EP 4 386 135 B1**

(12) **EUROPEAN PATENT SPECIFICATION**

(45) Date of publication and mention of the grant of the patent:
**24.06.2026 Bulletin 2026/26**

(21) Application number: **22855751.8**

(22) Date of filing: **28.06.2022**

(51) International Patent Classification (IPC):
**D21H 11/18** (2006.01)     **C08B 1/00** (2006.01)
**A61K 8/73** (2006.01)     **C08B 1/08** (2006.01)

(52) Cooperative Patent Classification (CPC):
**C08B 1/00; C08B 1/08; D21H 11/18; D21H 11/20**

(86) International application number:
**PCT/JP2022/025744**

(87) International publication number:
**WO 2023/017687 (16.02.2023 Gazette 2023/07)**

(54) **TYPE II UNMODIFIED CELLULOSE MICROFIBERS, AND METHOD FOR MANUFACTURING TYPE II UNMODIFIED CELLULOSE MICROFIBERS AND COMPACT OF SAME**

MIKROFASERN AUS UNMODIFIZIERTER CELLULOSE VOM TYP II UND VERFAHREN ZUR HERSTELLUNG VON MIKROFASERN AUS UNMODIFIZIERTER CELLULOSE VOM TYP II UND PRESSLING DARAUS

MICROFIBRES DE CELLULOSE NON MODIFIÉE DE TYPE II, ET PROCÉDÉ DE FABRICATION DE MICROFIBRES DE CELLULOSE NON MODIFIÉE DE TYPE II ET ENSEMBLE COMPACT DE CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.08.2021 JP 2021130565**

(43) Date of publication of application:
**19.06.2024 Bulletin 2024/25**

(73) Proprietor: **Futamura Kagaku Kabushiki Kaisha Nakamura-ku Nagoya-shi Aichi 450-0002 (JP)**

(72) Inventors:
• **IWATA, Ippei Ogaki-shi Gifu 503-0932 (JP)**
• **YAMAZAKI, Asuka Ogaki-shi Gifu 503-0932 (JP)**

(74) Representative: **TBK Bavariaring 4-6 80336 München (DE)**

(56) References cited:
WO-A1-2008/010464     WO-A1-2019/111933
JP-A- 2004 536 155     JP-A- 2015 157 796

• WANG HAIYING ET AL: "Effect of delignification technique on the ease of fibrillation of cellulose II nanofibers from wood", CELLULOSE, SPRINGER NETHERLANDS, NETHERLANDS, vol. 25, no. 12, 28 September 2018 (2018-09-28), pages 7003 - 7015, XP036632662, ISSN: 0969-0239, [retrieved on 20180928], DOI: 10.1007/S10570-018-2054-2
• SHARMA SUDHIR ET AL: "Characterization of micro fibrillation process of cellulose and mercerized cellulose pulp", RSC ADVANCES, vol. 5, no. 77, 1 January 2015 (2015-01-01), GB, pages 63111 - 63122, XP093289693, ISSN: 2046-2069, DOI: 10.1039/C5RA09068G

## Description

FIELD

The present invention relates to a manufacturing method for a cellulose fine fiber, particularly a manufacturing method for a type II unmodified cellulose fine fiber to obtain a chemically unmodified cellulose fine fiber.

BACKGROUND

In recent years, more attention has been placed on the international goals called Sustainable Development Goals (SDGs), established by the United Nations for sustainable development, among which environmental issues include reduction in the use amount of plastics. Efforts have aimed to address climate change through GHG emissions reduction by reducing the use amount of petroleum-derived plastics.

For example, microplastics (beads) are sometimes used in cosmetics such as foundations for the purpose of improving miscibility with other components and smoothness and texture during use. However, microplastics (beads) have been particularly raised as one of the causes of aggravating marine environmental pollution, and efforts have aimed at controlling the generation of and recovering microplastics.

For these reasons, alternatives for microplastics (beads) have been progressing in the market. However, it is known that particularly, microplastics (beads)-like fine resin raw materials having a particle size of 100 $\mu$m or less have not been widely used as alternative materials, and supply is small despite increasing demand.

Cellulose, which is a natural material and has biodegradability, has attracted attention as an alternative raw material to microplastics. Particularly, cellulose fine fibers such as cellulose nanofibers and cellulose microfibers can be processed into molded bodies and into beads and films, and are expected to be an alternative raw material to microplastics.

Examples of manufacturing methods for cellulose fine fibers include a method of subjecting a cellulose to an oxidation treatment in water using a catalyst and defibrating the resulting oxidized cellulose to obtain a cellulose nanofiber dispersion (refer to PTL 1), a method of carrying out carboxymethylation of cellulose in a mixed solvent of water and an organic solvent and defibrating the resulting carboxymethylated cellulose to obtain a dispersion of carboxymethylated cellulose nanofibers having a high degree of transparency (refer to PTL 2), and a manufacturing method of subjecting an anion-modified cellulose nanofiber salt to a desalting treatment by carrying out a cation exchange reaction using a cation exchange resin to obtain anion-modified cellulose nanofibers (refer to PTL 3).

WANG HAIYING ET AL: "Effect of delignification technique on the ease of fibrillation of cellulose II nanofibers from wood", CELLULOSE, SPRINGER NETHERLANDS, vol. 25, no. 12, 28 September 2018 discloses treatment of delignified wood pulp with 17.5 wt% NaOH to mercerize the cellulose, followed by grinding to obtain cellulose II nanofibers.

[CITATION LIST]

[PATENT LITERATURE]

[PTL 1] Japanese Unexamined Patent Publication (Kokai) No. 2008-001728
[PTL 2] Japanese Unexamined Patent Publication (Kokai) No. 2019-99758
[PTL 3] WO 2019/059079

SUMMARY

[TECHNICAL PROBLEM]

To obtain cellulose fibers which are type I cellulose fine fibers having a small fiber diameter, the cellulose fine fibers obtained by the above manufacturing methods are subjected to chemical defibration and mechanical (physical) defibration to form chemically modified cellulose. Since defibration is carried out using chemical agents, not only is a chemical removal step necessary, leading to step complication, but there are problems of limited applications, such as the inability to be used in cosmetics, and safety and environmental burden of the agents used.

The present inventors have repeatedly examined and improved upon manufacturing methods that do not use chemical defibration among manufacturing methods for obtaining a cellulose fine fiber having a small fiber diameter. As a result, the present inventors have completed a manufacturing method in which a chemically unmodified cellulose fine fiber can be more easily and efficiently obtained.

The present invention has been made in view of the above points and provides a cellulose fine fiber having a type II crystal structure obtained via mercerization of a cellulose and manufacturing methods for the cellulose fine fiber and a molded body thereof, wherein a chemically unmodified cellulose fine fiber having high transparency and safety can be efficiently obtained by simple steps.

[SOLUTION TO PROBLEM]

The first invention relates to a manufacturing method for a type II unmodified cellulose fine fiber, comprising: a mercerization step of mercerizing a cellulose to obtain a mercerized cellulose; a depolymerization step of reducing a degree of polymerization of the mercerized cellulose to 760 or less to obtain a raw cellulose; a defibration step of defibrating the raw cellulose by adding an alkali metal hydroxide so as to achieve a total concentration of 2.5 to 17.5% to obtain a cellulose fine fiber; and a neutralization step of neutralizing the cellulose fine fiber with an acid.

The second invention relates to the manufacturing method for a type II unmodified cellulose fine fiber according to the first invention comprising a molding step of molding the type II unmodified cellulose fine fibers into a molded product.

The third invention relates to a type II unmodified cellulose fine fiber obtained by the manufacturing method of the first invention, wherein a 0.1% by mass dispersion liquid of the type II unmodified cellulose fine fibers has a haze value of 35% or less measured in accordance with JIS K 7136 (2000).

The fourth invention relates to the type II unmodified cellulose fine fiber of the third invention, wherein a degree of polymerization of the type II unmodified cellulose fine fiber is 310 or less.

[ADVANTAGEOUS EFFECTS OF INVENTION]

According to the manufacturing method for a type II unmodified cellulose fine fiber according to the first invention, which comprises a mercerization step of mercerizing a cellulose to obtain a mercerized cellulose; a depolymerization step of reducing a degree of polymerization of the mercerized cellulose to 760 or less to obtain a raw cellulose; a defibration step of defibrating the raw cellulose by adding an alkali metal hydroxide so as to achieve a total concentration of 2.5 to 17.5% to obtain a cellulose fine fiber; and a neutralization step of neutralizing the cellulose fine fiber with an acid, a chemically unmodified cellulose fine fiber can be efficiently obtained by simple steps.

According to the manufacturing method for a type II unmodified cellulose fine fiber molded product according to the second invention, a molding step of molding type II unmodified cellulose fine fibers obtained by the manufacturing method for a type II unmodified cellulose fine fiber of the first invention to obtain a type II unmodified cellulose fine fiber molded product is included. The type II unmodified cellulose fine fiber molded product is useful as an alternative to plastic molded products.

According to the type II unmodified cellulose fine fiber according to the third invention, a 0.1% by mass dispersion liquid of the type II unmodified cellulose fine fibers obtained by the manufacturing method for a type II unmodified cellulose fine fiber of the first invention has a haze value of 35% or less measured in accordance with JIS K 7136 (2000). The type II unmodified cellulose fine fiber has high transparency, excellent appearance characteristics, and high safety, and thus can be used in a wide range of applications such as cosmetics.

According to the type II unmodified cellulose fine fiber according to the fourth invention, the type II unmodified cellulose fine fiber of the third invention has a degree of polymerization of 310 or less. Thus, the viscosity of the dispersion liquid of the cellulose fine fibers can be reduced, defoaming is facilitated, appearance during molding is improved, pressure increase in the molding apparatus can be suppressed, and production efficiency can be improved.

BRIEF DESCRIPTION OF DRAWINGS

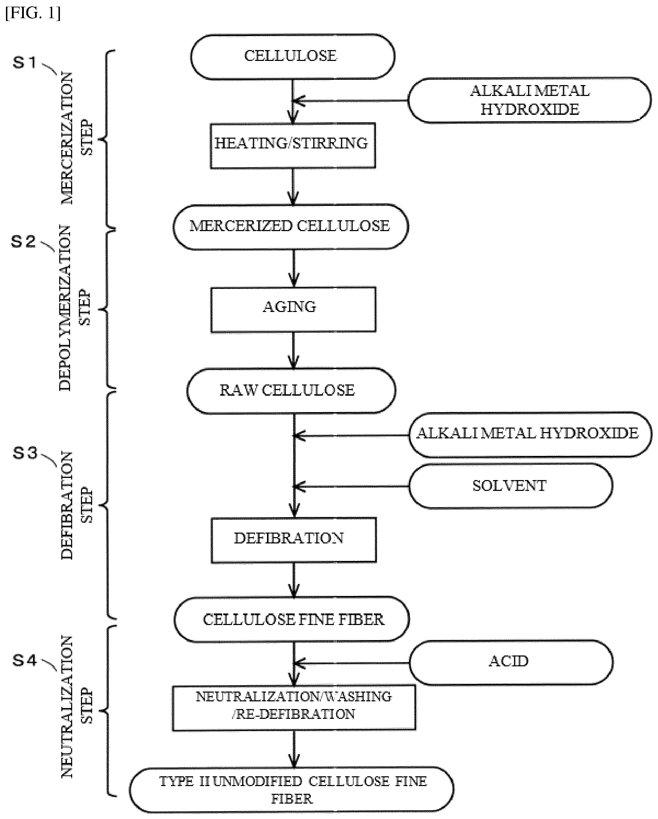

FIG. 1 is an outline process diagram according to the manufacturing method for a type II unmodified cellulose fine fiber of the present invention.

DESCRIPTION OF EMBODIMENTS

The cellulose fine fiber manufactured by the manufacturing method of the present invention is made into a fine fiber via a mercerization step of mercerizing a cellulose, and is thus a type II cellulose fine fiber having a type II crystal structure. Cellulose fine fibers are generally used as a reinforcement material for resins, and in such cases, type I cellulose fine fibers having high strength and a type I crystal structure are preferably used. Among cellulose fine fibers, type II cellulose fine fibers are softer than type I cellulose fine fibers, and are thus suitable since the addition thereof to cosmetics achieves a satisfactory texture.

The manufacturing method of the present invention aims at cellulose fine fibers as an alternative to petroleum-derived plastics in the field of cosmetics, and thus the cellulose fine fibers do not require the same strength characteristics as type I cellulose fibers used in applications of reinforcing materials contained in resins. With the expectation of a wide range of applications that require designability and satisfactory appearance characteristics such as cosmetics, the dispersion liquid of the cellulose fine fibers manufactured by the manufacturing method of the present invention has excellent transparency and high safety from the lack of chemical modification, and further exhibits satisfactory handling and excellent moldability.

The manufacturing method for a type II unmodified cellulose fine fiber of the present invention will be described sequentially using the process diagram of FIG. 1. First, as a starting material, the cellulose is preferably pulp. Pulp is a raw material that is mainly obtained by pulverizing wood and removing impurities such as lignin to increase purity of the cellulose component. Cotton linter pulp, which is obtained by removing impurities from cotton to increase purity of the cellulose component, is also used. In addition, pulp is fibrous, and is thus highly reactive with chemicals and preferable as a cellulose raw material. Other than pulp, animal celluloses such as bacterial celluloses produced by microorganisms can be used. A purified cellulose obtained by purifying any of the above raw materials can be used.

A mercerization step (S1) is a step of mercerizing a cellulose to obtain a mercerized cellulose. In the mercerization step, a cellulose as a raw material is added to an alkali metal hydroxide such as caustic soda (NaOH), and if necessary, the mixture is stirred while heated to swell a cellulose fiber. When a cellulose fiber is immersed in the alkali metal hydroxide, the cellulose fiber is negatively charged and generates a Coulomb force. Fibers repel each other, which facilitates defibration. Since the mercerized cellulose is easily defibrated as described above, the energy required in the subsequent defibration step can be reduced.

Examples of the alkali metal hydroxide used in the mercerization step include caustic soda (NaOH), lithium hydroxide, and potassium hydroxide. From the viewpoints of cost, safety, and environmental burden, caustic soda is preferably used.

After the mercerization step (S1), excess alkali metal hydroxide is removed if necessary. A depolymerization step (S2) is carried out to appropriately adjust the solid content concentration. The depolymerization step (S2) is a step of reducing a degree of polymerization of the mercerized cellulose obtained from the mercerization step (S1) to 760 or less. The mercerized cellulose having the solid content thereof adjusted is appropriately pulverized and aged by oxidative decomposition with oxygen in the air to reduce the degree of polymerization. At this time, the degree of polymerization is set to 760 or less. When the degree of polymerization of the mercerized cellulose is 760 or less, the transparency of the resulting cellulose fine fiber dispersion liquid is ensured. The lower the degree of polymerization of the mercerized cellulose, the easier the defibration of cellulose fibers in the later defibration step.

The aging of the mercerized cellulose in the depolymerization step (S2) is carried out at room temperature or under heating conditions. To speed up the depolymerization rate, heating conditions are preferably used so that the raw material is not dried. An aging accelerator such as manganese (II) sulfate, which accelerates the aging reaction, can also be added.

A raw cellulose that can be defibrated into fine fibers is obtained via the mercerization step (S1) and the depolymerization step (S2). By mercerizing the cellulose, the raw cellulose becomes a type II cellulose having a type II crystal structure. A type II cellulose is said to be inferior to a type I cellulose in terms of strength. However, the cellulose fine fiber obtained by the present invention is intended to be used as an alternative to plastics in fields such as cosmetics and does not require strength as high as that of a type I cellulose. Thus, the decrease in strength does not pose a problem.

In a defibration step (S3), an alkali metal hydroxide and a solvent (deionized water) are added to the raw cellulose to adjust the total concentration to 2.5 to 17.5%, and the raw cellulose is subjected to defibration. As described above, examples of the alkali metal hydroxide used herein include caustic soda, lithium hydroxide, and potassium hydroxide. Caustic soda is preferably used from the viewpoints of cost and safety. Defibration of the raw cellulose is carried out by mechanical (physical) defibration. Mechanical (physical) defibration is carried out by a known method using a homogenizer or a water jet. The raw cellulose is in a state where fibers are swollen by mercerization and easily defibrated and the degree of polymerization is reduced by the depolymerization step, and thus can be easily defibrated without applying high pressure, which is also significant in terms of equipment.

When the concentration of the alkali metal hydroxide is lower than 2.5%, the swelling of the cellulose is insufficient and defibration may be difficult. When the concentration of the alkali metal hydroxide is higher than 17.5%, salt concentration increases and cellulose fibers tend to agglomerate, which may hinder defibration. If the content of the alkali metal hydroxide is outside of the above range and defibration is insufficient, the resulting cellulose fine fiber dispersion liquid has low transparency and poor designability.

The defibration may be carried out in multiple steps. For example, after a preliminary defibration by a mixer, a main defibration is carried out using a homogenizer, whereby uniform cellulose fine fibers having a small fiber diameter can be obtained. From the preliminary defibration, problems such as clogging of the defibration apparatus by the raw cellulose can be avoided. The preliminary defibration is carried out by a known method using a mixer or a refiner. In the defibration of cellulose fine fibers, the defibration preferably results in an average fiber diameter from nano-size to hundreds of nano-size. When the average fiber diameter is about 2 to 800 nm, preferably 100 nm or less, the transparency of the cellulose fine fiber dispersion liquid is improved.

The cellulose fine fiber obtained via the defibration step is neutralized with an acid in a neutralization step (S4). The cellulose fine fiber obtained via the defibration step is strongly alkaline and thus requires neutralization. Examples of acids used include sulfuric acid, hydrochloric acid, and lactic acid. The neutralized cellulose fine fiber is appropriately washed and re-defibrated to obtain a type II unmodified cellulose fine fiber.

A molded body can be produced by using a dispersion liquid of the type II unmodified cellulose fine fibers obtained via the above steps. For example, the dispersion liquid can be made into a film by coating film formation, or can be made into beads for use in cosmetics. Both can be produced by a dry molding step. Compared to conventional cellulose films and cellulose beads, the amount of chemicals that impose a high environmental burden can be reduced.

A 0.1% by mass dispersion liquid of the type II unmodified cellulose fine fibers obtained by the manufacturing method of the present invention has satisfactory transparency. Specifically, when the haze value thereof measured in accordance with JIS K 7136 (2000) is 35% or less, the dispersion liquid has a satisfactory appearance when made into a film, and can be used in cosmetics and a wide range of applications.

By setting the degree of polymerization of the type II unmodified cellulose fine fibers obtained by the manufacturing method of the present invention to 310 or less, the viscosity of the dispersion liquid can be reduced, defoaming is facilitated, which improves moldability, and appearance of the molded product is improved. Further, when the viscosity of the dispersion liquid is reduced, pressure increase in the molding apparatus can be suppressed, and production efficiency can be improved.

EXAMPLES

In the manufacture of type II unmodified cellulose fine fibers, the present inventors used the raw materials below, followed the process diagram of FIG. 1, and changed each of the degree of polymerization in the depolymerization step and the alkali metal hydroxide concentration in the defibration step to carry out manufacturing experiments of the type II unmodified cellulose fine fibers.

[Raw material]

For the cellulose raw material, which is the starting raw material, dissolving pulp ("LNDP" manufactured by Nippon Paper Industries Co., Ltd.) was used.

[Alkali metal hydroxide]

For the alkali metal hydroxide used for mercerization in the mercerization step, caustic soda (manufactured by Kishida Chemical Co., Ltd.) was used. The same caustic soda was used in the defibration step.

[Acid]

For the acid in the neutralization step, sulfuric acid (manufactured by Kishida Chemical Co., Ltd.) was used.

[Preparation of dispersion liquid of type II unmodified cellulose fine fibers]

A dispersion liquid of type II unmodified cellulose fine fibers was prepared according to the following formulation using the above raw materials.

<Prototype Example 1>

18% by weight of caustic soda was heated to 50 °C, pulp was charged therein so as to reach 2% by weight and stirred until the mixture became a slurry, and the slurry was mercerized (mercerization step). Excess caustic soda was then removed to adjust the solid content to 33% by weight. The mercerized cellulose was aged at 50 °C to obtain a raw cellulose 1 having a degree of polymerization of 752 (depolymerization step). 10.6 g of the raw cellulose 1, 330.65 g of deionized water, and 8.75 g of caustic soda (total caustic soda concentration of 2.5%) were then charged into a 500-mL container and mixed with a mixer ("Labolution" manufactured by Primix Corporation) to carry out a preliminary defibration. A main defibration was then carried out with a homogenizer ("LAB 1000" manufactured by SMT Co., Ltd.) (defibration step). 125 g of the prepared slurry was sampled and neutralized by adding 20% by weight of sulfuric acid while stirring (neutralization step). The neutralized sample was subjected to suction filtration and then displacement washing with 300 mL of deionized water. Deionized water was added to the washed sample so that the total weight was 250 g, and a preliminary defibration was carried out using a mixer ("Labolution" manufactured by Primix Corporation). The fibers were then defibrated with a homogenizer ("LAB 1000" manufactured by SMT Co., Ltd.) to obtain a dispersion liquid of type II unmodified cellulose fine fibers of Prototype Example 1.

<Prototype Example 2>

Except that the total concentration of caustic soda in the defibration step was set to 9.5%, a dispersion liquid of the type II unmodified cellulose fine fibers of Prototype Example 2 was obtained in the same manner as in Prototype Example 1.

<Prototype Example 3>

Except that the total concentration of caustic soda in the defibration step was set to 17.5%, a dispersion liquid of the type II unmodified cellulose fine fibers of Prototype Example 3 was obtained in the same manner as in Prototype Example 1.

<Prototype Example 4>

Except that the total concentration of caustic soda in the defibration step was set to 1.5%, the same method as in Prototype Example 1 was carried out. However, the cellulose was not defibrated and a dispersion liquid of the type II unmodified cellulose fine fiber could not be obtained.

<Prototype Example 5>

Except that the total concentration of caustic soda in the defibration step was set to 18.5%, a dispersion liquid of the type II unmodified cellulose fine fibers of Prototype Example 5 was obtained in the same manner as in Prototype Example 1.

<Prototype Example 6>

Except that the aging treatment was carried out until the degree of polymerization of the mercerized cellulose reached 299 in the depolymerization step to obtain a raw cellulose, a dispersion liquid of the type II unmodified cellulose fine fibers of Prototype Example 6 was obtained in the same manner as in Prototype Example 1.

<Prototype Example 7>

Except that the aging treatment was carried out until the degree of polymerization of the mercerized cellulose reached 299 in the depolymerization step to obtain a raw cellulose, a dispersion liquid of the type II unmodified cellulose fine fibers of Prototype Example 7 was obtained in the same manner as in Prototype Example 2.

<Prototype Example 8>

Except that the aging treatment was carried out until the degree of polymerization of the mercerized cellulose reached 299 in the depolymerization step to obtain a raw cellulose, a dispersion liquid of the type II unmodified cellulose fine fibers of Prototype Example 8 was obtained in the same manner as in Prototype Example 3.

<Comparative Example 1>

Except that the depolymerization step was not carried out (was omitted), the same method as in Prototype Example 1 was carried out. However, the cellulose was not defibrated and a dispersion liquid of the type II unmodified cellulose fine fibers could not be obtained.

<Comparative Example 2>

Except that the depolymerization step was not carried out (was omitted), a dispersion liquid of the type II unmodified cellulose fine fibers of Comparative Example 2 was obtained in the same manner as in Prototype Example 3.

<Comparative Example 3>

Except that the mercerization step and the depolymerization step were not carried out (were omitted), the same method as in Prototype Example 1 was carried out. However, the cellulose was not defibrated and a dispersion liquid of the type II unmodified cellulose fine fibers could not be obtained.

Haze (%) and degree of polymerization were measured for the dispersion liquid of type II unmodified cellulose fine fibers of each Prototype Example and Comparative Example. The degree of polymerization of each raw cellulose and the type and concentration (%) of alkali metal hydroxide in the defibration step for each Prototype Example and Comparative Example are shown in Table 1.

[Haze]

Haze (%) is an index of transparency and was measured for the 0.1% by mass dispersion liquid of each Prototype Example using a haze meter (NDH-4000 manufactured by Nippon Denshoku Industries Co., Ltd.) in accordance with JIS K 7136 (2000). Deionized water was used to adjust the concentration of the dispersion liquid in each Prototype Example. The dispersion liquid was added into a glass cell (MG-40 manufactured by Fujiwara Scientific Co., Ltd.) for liquids having a light path of 1 cm and measured. Zero-point measurement was carried out by adding deionized water into the same glass cell. The Prototype Examples and Comparative Examples in which cellulose fine fibers could not be obtained due to failure in defibration of cellulose and thus could not be measured were indicated as "-".

[Degree of polymerization]

Degree of polymerization was measured by the following method, a viscosity method using a copper ethylenediamine solution. Dried cellulose fine fibers were dissolved in a 0.5 M copper ethylenediamine solution 1 to form a solution 2. The viscosities of the solution 1 and the solution 2 were measured using a capillary viscometer. Designating the viscosity of the solution 2 as $\eta$ and the viscosity of the solution 1 as $\eta_0$ the limiting viscosity $[\eta]$ of the cellulose fine fibers and the degree of polymerization DP were determined by the following formula. c is the concentration of cellulose fine fibers (g/L).

$$\text{Limiting viscosity } [\eta] = \{(\eta/\eta_0) - 1\}/c$$

$$\text{Degree of polymerization DP} = \text{limiting viscosity } [\eta]/(8.8 \times 10^{-4})$$

For the Prototype Examples and Comparative Examples in which cellulose fine fibers could not be obtained due to failure in defibration of cellulose, the degree of polymerization of the dispersion liquid of cellulose fine fibers could not be measured.

[Average fiber diameter]

The average fiber diameter was obtained by measuring the diameters of 50 or more fibers in a scanning range of 10-$\mu$m square using a scanning probe microscope (SPM-9700HT manufactured by Shimadzu Corporation) and calculating the average value thereof. Samples for scanning probe microscope observation were prepared by diluting a dispersion liquid of cellulose fine fibers with water to an arbitrary concentration and casting the diluted dispersion liquid on a mica substrate, followed by air-drying. The average fiber diameter was measured only for Prototype Example 7.

[Table 1]

| | Degree of polymerization (raw cellulose) | Alkali metal hydroxide | | Haze (%) | Degree of polymerization (dispersion liquid) | Average fiber diameter (nm) |
| --- | --- | --- | --- | --- | --- | --- |
| | | Type | Concentration (%) | | | |
| Prototype Example 1 | 752 | NaOH | 2.5 | 33.4 | 302 | - |
| Prototype Example 2 | 752 | NaOH | : 9.5 | 4.1 | 188 | - |
| Prototype Example 3 | 752 | NaOH | 17.5 | 27.7 | 261 | - |
| Prototype Example 4 | 752 | NaOH | 1.5 | - | - | - |
| Prototype Example 5 | 752 | NaOH | 18.5 | 67.8 | 309 | - |
| Prototype Example 6 | 299 | NaOH | : 2.5 | 20.5 | 180 | - |

(continued)

| | Degree of polymerization (raw cellulose) | Alkali metal hydroxide | | Haze (%) | Degree of polymerization (dispersion liquid) | Average fiber diameter (nm) |
|---|---|---|---|---|---|---|
| | | Type | Concentration (%) | | | |
| Prototype Example 7 | 299 | NaOH | 9.5 | 2.2 | 159 | 5.92 |
| Prototype Example 8 | 299 | NaOH | 17.5 | 14.7 | 171 | - |
| Comparative Example 1 | 1011 | NaOH | 2.5 | - | - | - |
| Comparative Example 2 | 1011 | NaOH | 17.5 | 37.9 | 302 | - |
| Comparative Example 3 | 1006 | NaOH | 2.5 | - | - | - |

[Results and observations]

When Prototype Examples 1 and 6 and Comparative Example 1 having the same alkali metal hydroxide concentration were compared with Prototype Examples 3 and 8 and Comparative Example 2, it was shown that the lower the degree of polymerization of a raw cellulose, the lower the haze of the cellulose fine fiber dispersion liquid. From this, it was shown that by obtaining a raw cellulose in which the degree of polymerization of the mercerized cellulose is further reduced in the depolymerization step, cellulose fine fibers could be dispersed more finely and uniformly with the same defibration energy. In Comparative Example 1, when a raw cellulose having a degree of polymerization of 760 or greater was obtained without undergoing the depolymerization step, the cellulose could not be defibrated if the alkali metal hydroxide concentration in the defibration step was low. In Comparative Example 2, even when defibration was achieved by increasing the concentration of alkali metal hydroxide, the resulting dispersion had a large haze.

When Prototypes Examples 1 to 5 having the same degree of polymerization of the raw cellulose were compared, it was shown that the haze of the cellulose fine fiber dispersion liquid was increased when the concentration of alkali metal hydroxide was too low or too high. Particularly, in Prototype Example 4, when the alkali metal hydroxide concentration was less than 2.5%, swelling of the cellulose fibers was insufficient and defibration was not possible, leading to failure to obtain cellulose fine fibers. Further, it was considered that in Prototype Example 5, when the alkali metal hydroxide concentration is higher than 17.5%, salt concentration increased and cellulose fibers aggregated, defibration of cellulose was insufficient, and haze of the dispersion liquid was increased. In other words, it was shown that a dispersion liquid having a low haze can be obtained by setting the concentration of alkali metal hydroxide in the defibration step to 2.5 to 17.5%. Particularly, it was found that when the total concentration is about 10%, a dispersion liquid having a higher transparency can be obtained.

In Comparative Example 3, which did not undergo the mercerization step, since the cellulose had a type I crystal structure and the degree of polymerization of the raw cellulose was high due to omission of the depolymerization step, defibration could not be carried out and cellulose fine fibers could not be obtained.

From the foregoing, it was shown that according to the manufacturing method of the present invention, a dispersion liquid of cellulose fine fibers having low haze and high transparency can be obtained even with a small defibration energy, and thus a chemically unmodified type II cellulose fine fiber can be manufactured efficiently by simple steps.

INDUSTRIAL APPLICABILITY

According to the manufacturing method for a type II unmodified cellulose fine fiber of the present invention, a chemically unmodified cellulose fine fiber can be efficiently obtained by simple steps. In addition, the resulting type II unmodified cellulose fine fiber has high transparency, low viscosity, easy handling, and excellent appearance characteristics, and thus can be used in a wide range of applications such as cosmetics and is useful as an alternative to plastics.

REFERENCE SIGNS LIST

S1    mercerization step
S2    depolymerization step
S3    defibration step
S4    neutralization step

**Claims**

1. A manufacturing method for a type II unmodified cellulose fine fiber, comprising:

   a mercerization step of mercerizing a cellulose to obtain a mercerized cellulose;
   a depolymerization step of reducing a degree of polymerization of the mercerized cellulose to 760 or less to obtain a raw cellulose;
   a defibration step of defibrating the raw cellulose by adding an alkali metal hydroxide so as to achieve a total concentration of 2.5 to 17.5% to obtain a cellulose fine fiber; and
   a neutralization step of neutralizing the cellulose fine fiber with an acid.

2. The manufacturing method for a type II unmodified cellulose fine fiber according to claim 1 comprising a molding step of molding the type II unmodified cellulose fine fibers into a molded product.

3. A mercerized, defibrated, and neutralized type II unmodified cellulose fine fiber having a degree of polymerization of 760 or less obtained by the manufacturing method according to claim 1, wherein a 0.1% by mass dispersion liquid of the type II unmodified cellulose fine fibers has a haze value of 35% or less measured in accordance with JIS K 7136 (2000).

4. The type II unmodified cellulose fine fiber according to claim 3, wherein a degree of polymerization of the type II unmodified cellulose fine fiber is 310 or less.

**Patentansprüche**

1. Verfahren zur Herstellung einer unmodifizierten Typ-II-Zellulose-Feinfaser/, umfassend:

   einen Merzerisierungsschritt, bei dem eine Zellulose merzerisiert wird, um eine merzerisierte Zellulose zu erhalten;
   einen Depolymerisationsschritt, bei dem ein Polymerisationsgrad der merzerisierten Zellulose auf 760 oder weniger verringert wird, um einen Rohzellstoff zu erhalten;
   einen Defibrierungsschritt zum Defibrieren des Rohzellstoffes durch Zugabe eines Alkalimetallhydroxids, um eine Gesamtkonzentration von 2,5 bis 17,5 % zu erreichen, um eine Zellulose-Feinfaser zu erhalten; und
   einen Neutralisationsschritt zum Neutralisieren der Zellulose-Feinfaser mit einer Säure.

2. Verfahren zur Herstellung einer unmodifizierten Typ-II-Zellulose-Feinfaser gemäß Anspruch 1, umfassend einen Formungsschritt zum Formen der unmodifizierten Typ-II-Zellulose-Feinfasern zu einem Formprodukt.

3. Merzerisierte, defibrierte und neutralisierte unmodifizierte Typ-II-Zellulose-Feinfaser mit einem Polymerisationsgrad von 760 oder weniger, erhalten durch das Herstellungsverfahren gemäß Anspruch 1, wobei eine 0,1-Massen-% Dispersionsflüssigkeit der unmodifizierten Typ-II-Zellulose-Feinfasern einen Trübungswert von 35 % oder weniger aufweist, gemessen gemäß JIS K 7136 (2000).

4. Unmodifizierte Typ-II-Zellulose-Feinfaser gemäß Anspruch 3, wobei ein Polymerisationsgrad der unmodifizierten Typ-II-Zellulose-Feinfaser 310 oder weniger ist.

**Revendications**

1. Procédé de fabrication d'une fibre fine de cellulose non modifiée de type II, comprenant :

   une étape de mercerisation consistant à merceriser une cellulose pour obtenir une cellulose mercerisée ;
   une étape de dépolymérisation consistant à réduire un degré de polymérisation de la cellulose mercerisée à 760 ou moins pour obtenir une cellulose brute ;
   une étape de défibrage consistant à défibrer la cellulose brute en ajoutant un hydroxyde de métal alcalin de manière à atteindre une concentration totale de 2,5 à 17,5 % afin d'obtenir une fibre fine de cellulose ; et
   une étape de neutralisation consistant à neutraliser la fibre fine de cellulose avec un acide.

**2.** Procédé de fabrication d'une fibre fine de cellulose non modifiée de type II selon la revendication 1, comprenant une étape de moulage consistant à mouler les fibres fines de cellulose non modifiées de type II en un produit moulé.

**3.** Fibre fine de cellulose non modifiée de type II, mercerisée, défibrée et neutralisée, ayant un degré de polymérisation de 760 ou moins et obtenue par le procédé de fabrication selon la revendication 1, dans laquelle une dispersion liquide à 0,1 % en masse des fibres fines de cellulose non modifiées de type II a une valeur de trouble de 35 % ou moins, mesurée conformément à la norme JIS K 7136 (2000).

**4.** Fibre fine de cellulose non modifiée de type II selon la revendication 3, dans laquelle un degré de polymérisation de la fibre fine de cellulose non modifiée de type II est de 310 ou moins.

[FIG. 1]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008001728 A
- JP 2019099758 A
- WO 2019059079 A

**Non-patent literature cited in the description**

- Effect of delignification technique on the ease of fibrillation of cellulose II nanofibers from wood. **WANG HAIYING et al.** CELLULOSE. SPRINGER NETHERLANDS, 28 September 2018, vol. 25